# EUROPEAN PATENT SPECIFICATION

(11) **EP 2 260 304 B1**
(45) Date of publication and mention of the grant of the patent: **01.02.2012**
(21) Application number: 09731266.4
(22) Date of filing: 06.04.2009
(51) Int. Cl.: G01N 33/574

(54) **METHODS FOR DETECTING TUMOR-SPECIFIC FUSION PROTEINS**
VERFAHREN ZUM NACHWEIS VON TUMORSPEZIFISCHEN FUSIONSPROTEINEN
PROCÉDÉS DE DÉTECTION DE PROTÉINES DE FUSION SPÉCIFIQUES DES TUMEURS

(30) Priority: 07.04.2008 EP 08154164; 07.04.2008 US 123369 P
(43) Date of publication of application: 15.12.2010
(73) Proprietor: Erasmus University Medical Center Rotterdam, 3015 GE Rotterdam (NL)
(72) Inventor: VAN DONGEN, Jacobus Johannes Maria, NL-2914 LE Nieuwerkerk aan den IJssel (NL)
(74) Representative: Habets, Winand
(86) International application number: PCT/EP2009/054072
(87) International publication number: WO 2009/124901

(56) References cited:
- EP-A- 1 507 147
- WO-A-00/50903
- WO-A-02/054074
- WO-A-2007/061684
- US-A1- 2007 015 134
- LATGER V ET AL: "PATHOLOGIE VASCULAIRE ET ACTIVATION CELLULAIRE. EXPLORATION DU PHENOTYPE CELLULAIRE ADHERENT PAR CYTOMETRIE EN FLUX QUANTITATIVE" JOURNAL DES MALADIES VASCULAIRES, MASSON, PARIS, FR, vol. 24, no. 1, 1 January 1999 (1999-01-01), pages 11-18, XP000910966 ISSN: 0398-0499
- MARTINEZ NATALIA ET AL: "The oncogenic fusion protein RUNX1-CBFA2T1 supports proliferation and inhibits senescence in t(8;21)-positive leukaemic cells" BMC CANCER, BIOMED CENTRAL, LONDON, GB, vol. 4, no. 1, 6 August 2004 (2004-08-06), page 44, XP021004644 ISSN: 1471-2407 cited in the application

## Description

### Field of the invention

The invention relates to the field of cancer diagnosis and the application of diagnostic techniques in pathology and hematology. Specifically, the invention relates to flow cytometric techniques, for the detection of tumor specific gene products exclusively expressed by tumor cells containing chromosomal aberrations, wherein a sample is contacted with at least two different probes directed against the fusion protein, each probe being reactive with a distinct site on the fusion protein.

### Background of the invention

Detection methods of this type are known in the art. For example, US 6,686,165 describes a rapid multiplexed, bead-based immunoassay for simultaneous detection of multiple fusion proteins by flow cytometry. Fusion protein-specific monoclonal antibodies (catching probes) can be covalently coupled to size- or color-coded beads. The antibody-coupled beads can be used to capture a fusion protein in the sample, and the bound fusion proteins are then detected with a cocktail of fluorochrome-conjugated, fusion protein-specific antibodies (detection probes) using flow cytometry. By utilizing appropriate size- or color-coded beads, the multiplexed immunoassay can be readily formatted into a single-tube assay for simultaneously detecting several fusion proteins within one disease category, e.g. acute myeloid leukemia (AML) or precursor-B-ALL.

WO2005/015235 relates essentially to a further improvement of the method of US 6,686,165. It discloses that the sensitivity of a bead-based catching/detection assay as described in US 6,686,165 can be significantly improved by enriching a sample for fusion protein A-B relative to the native non-fused protein A and/or protein B prior to detecting the fusion protein. This is achieved by depleting a sample of one or more non-fused, native counterparts of the fusion protein.

The above mentioned fusion protein detection techniques have proven to be highly valuable for the diagnosis and monitoring of disease, in particular leukemia, lymphoma and solid tumours resulting from chromosomal aberrations that lead to fusion genes.

WO 2007/061684 is concerned with the detection of fusion gene products resulting from chromosomal translocations wherein antibody probes are used to detect both parts of a fusion protein.

WO 00/50903A, Lager et al., 1999, J. Maladies Vasculaires 24, 11-18 and US2007/015134 disclose the use of quantification beads characterized by a known amount of protein to be detected attached on their surface.

EP 1507147 A also describes a method for detecting low levels of a fusion protein in a sample.

WO 02/054074 deals with the recognition of tumor specific gene products in cancer using two different probes directed against the fusion protein.

However, it appears that there is a need for further improvements of the method. The present invention aims to fulfil this need. For example, there are known cases wherein detection of a tumor-specific fusion gene by PCR yields a positive test result, whereas detection of the fusion gene product at the protein level yielded a negative test result. In particular, it is an object of the present invention to minimize the number of overlooked false-negative outcomes of the fusion protein assay, as well as to reduce the incidence of false-negative assay outcomes. A further object is to provide a method that allows for a reliable quantitation of one or more fusion proteins. This is especially important in inter- and intra-patient monitoring, for example to evaluate a response to anti-cancer treatment. The invention preferably aims at resolving these issues simultaneously in a simple and convenient manner.

### Summary of the invention

It has been found that at least some of the goals can be met by performing, in addition to the fusion protein detection analysis, one or more additional qualitative and/or quantitative control analysis, involving the use of so-called "quality control beads" and/or quantitation beads". Further assay improvements relate to modifications of the sample preparation procedure, in particular the step of preparing a cell lysate to be assayed for the presence of one or more tumor-specific fusion proteins. As will become clear from the description below, the improvements disclosed herein are fully compatible with each other. They may be employed as such or in any combination with each other.

Described herein is a method for detecting a tumor-specific fusion protein, said method comprising (A) a fusion protein analysis wherein a lysate of cells suspected to contain at least one tumor-specific fusion protein is contacted with at least a bead-bound catching probe and a fluorochrome-conjugated detection probe directed against the at least one fusion protein, each probe capable of recognizing a binding site positioned at opposite sides of the fusion region of said fusion protein, and determining binding of said probes to said fusion protein by flow cytometry, the method furthermore comprising subjecting the lysate to (B) a qualitative control analysis and/or (C) a quantitative control analysis.

Step (B) comprises contacting the cell lysate with at least a bead-bound catching probe and a fluorochrome-conjugated detection probe directed against a household protein known to be present in the cells, each probe capable of recognizing distinct binding sites of said household protein, and determining binding of said probes to said household protein by flow cytometry to determine the amount of intact protein in the lysate being analyzed for the presence of a fusion protein.

Step (C) comprises contacting a fluorochrome-conjugated fusion protein detection probe as used in fusion protein analysis step (A) with at least a first set of quantitation beads, the beads being provided with a known amount of binding sites for the fluorochrome-conjugated fusion protein detection probe, and measuring by flow cytometry the fluorescence signal that is associated with said known amount of binding sites. The latter can provide an indication of the amount of fusion protein detected in step (A).

Thus, in addition to the fusion protein detection analysis (A), for example employing the teaching of US 6,686,165 or WO2005/015235, a method of the invention is characterized by a qualitative control analysis (B) and a quantitative control analysis (C). Preferably, a method of the invention comprises analysis (A) (B) and (C) simultaneously in a single tube.

### Detailed description of the invention

Analysis (B) is based on the insight that upon lysis of several types of cells that are of interest to be assayed for the presence of a tumor-specific fusion protein, in particular leukocytes, a protease activity is released, which can cause an unwanted protein degradation. Especially more mature myeloid cells appear to contain granules with high levels of various proteases, which rapidly degrade the cellular proteins that are released upon cell lysis. Also the released fusion proteins appear to be degraded upon cell lysis, if (mature) myeloid cells are present in the analyzed leukocyte sample. This protease problem is particularly prominent in case of BCR-ABL fusion protein detection in cell lysates of chronic myelogenous leukemia (CML) cells. CML cells represent more mature myeloid cells and consequently can contain high levels of proteases in their granules.

If the integrity of the proteins in the cell lysate is not checked, it might well be that false-negative results are obtained because the fusion protein will not be detectable because of protein degradation. Therefore, it is important to verify the integrity of the proteins in a cell lysate to be subjected to immunobead assays, especially if the cell sample contains (more) mature myeloid cells, such as granulocytes or CML cells.

Quality control analysis (B) is advantageously performed at the time of diagnosis, when a patient sample suspected to contain a tumor-specific fusion protein is analyzed for the presence of one or more fusion proteins known to be involved in malignancies. This is typically done using a multiplex format assay, wherein different sets of probes are used to simultaneously detect different fusion proteins. The use of quality control beads in analysis (B) ensures that a possible negative assay outcome, i.e. the absence of a tumor-specific fusion protein, is interpreted correctly in the sense that conclusions can only be drawn if the cell lysate is found to be sufficient quality in terms of protein integrity.

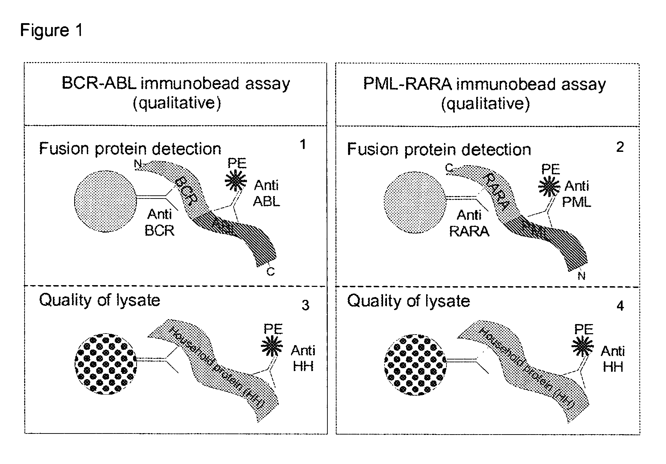

By designing a multiplex assay, e.g. using beads with different sizes and/or colours, the fusion protein and the household protein can be detected in a single tube (Figure 1). This guarantees an optimal quality control of the fusion protein detection assay. For example, if the signal of the household protein is found to be below a certain threshold level, a negative outcome of the fusion protein analysis (i.e. no detection of fusion protein) should be discarded or at least be verified by other detection methods, such as a PCR-based method for detecting the corresponding fusion gene. Alternatively, or in addition, the fusion protein analysis can be repeated using a sample of improved quality, for instance a lysate wherein protease activity is (further) inhibited and/or wherein nuclear fusion proteins are released from DNA. See herein below for details.

A household gene or a household protein is any gene or protein that is expressed in a cell irrespective of the expression of tumor-associated fusion proteins. Preferably, the household gene or protein is constitutively expressed. Also preferred is a household protein that is expressed in the cell types to be tested.

According to the invention, the integrity of the proteins in a cell lysate is evaluated by checking the integrity of a well-defined household protein that is stably present in the cells to be tested. For example, in leukocytes the household protein to be detected may be ABL, β2m, actin, MGUS, GADH, actin such as β-actin and the like. It is convenient to detect this household protein via a comparable immunobead assay as the fusion protein. Thus, a household protein can be detected using at least a bead-bound catching probe and a fluorochrome-conjugated detection probe directed against distinct binding sites on the household protein. Preferably, the two probes do not compete for binding to the household protein. Even more preferred the at least two household protein binding sites or epitopes that are recognized by the different probes should spatially be sufficiently separated from each other in order to have potential protease target sites between the two epitopes, so that a negative result is obtained in case of protease activity in the cell lysate. Therefore, in one embodiment the catching probe and the detection probe directed against a household protein are capable of recognizing, respectively, an N-terminal and a C-terminal binding site of said household protein, or, respectively, a C-terminal and a N-terminal binding site of said household protein. In order to obtain th ehighest sensitivity of the assay, it is preferred that the epitopes reside in a stretch of amino acids that make up the terminal 25%, preferably 15%, most preferably the terminal 10% of the household protein.

Probes against a certain epitope can be produced by methods known in the art. For example, specific (monoclonal) antibody probes can be generated using a recombinant protein domain or a peptide immunogen comprising the epitope.

The term 'probe' refers to any moiety that comprises a portion that can specifically bind a protein target to be detected in a method of the invention, i.e. a fusion protein or a household protein. Probes may include nucleic acids, peptides, PNAs and other molecules that can specifically bind to a target. Very suitable probes are (monoclonal) antibodies or functional fragments thereof.

The term 'tumor-specific fusion protein' is meant to refer to all known and yet to be discovered proteins encoded by malignant fusion genes. Tumor-specific fusion proteins are widely known in the art. Included are those mentioned in tables 1 and 2 of US 6,636,165. See also, the fusion proteins disclosed in Mitelman et al., Nat. Rev. Cancer 2007; 7:233-245 .

The term "fluorochrome" refers to any fluorescent molecule or moiety capable of generating a detectable signal. Fluorescent dyes useful to label a probe include fluoresceins, rhodamines, benzophenoxazines, energy-transfer dyes, and cyanines.

The term 'bead' refers to a particle that has the appropriate characteristics for analysis by flow cytometry. Such beads are known in the art. They may be made of any suitable material, including plastic, polystyrene, latex and other polymeric substances and are generally spherical. To allow for multiplex assays, the different types of beads (be it fusion protein catching beads, household protein catching beads and/or different sets of quantitation beads) may preferably be distinguishable from each other. For example, beads of different size and/or color may be used. The term "immunobead" as used herein refers to a bead to which an antibody probe is conjugated.

A further control analysis comprises a quantitative control analysis. Quantitation of the amount of fusion protein has so far not been used in applications of a bead-based assay for detecting tumor-specific fusion proteins. Tumor-specific fusion proteins can be used for, among others, the detection of minimal residual disease (MRD) and it would be desirable to provide a method for reliable quantitation of the fusion proteins. For this purpose an elegant bead-based assay can be applied, using multiple different sets of beads (e.g. two or more sets of differently colored beads) each of them covered with different densities of the protein domain that is recognized by the detection antibody of the involved immunobead assay (e.g. anti-ABL in case of the BCR-ABL assay). Accordingly, step (C) of a method of the invention preferably comprises the use of at least a first set of quantitation beads comprising a first known quantity of binding sites and a second set of quantitation beads comprising a second known quantity of said binding sites, to allow the plotting of a standard (or calibration) curve. As will be understood, a more reliable standard curve can be obtained using more than two sets of quantitation beads. In a preferred embodiment, a mixture of at least three sets of differently sized/colored beads is used, each set being provided with a different quantity and/or desity of binding sites. For example, a mixture of five sets of quantitation beads is included in the assay, to obtain a standard curve ranging from 300 to 30000 binding sites (antigens) per bead. The amount of beads within a set can vary. For example, as little as 50 or 100 beads may yield a sufficient signal.

Beads can be provided with a known amount of binding sites by methods known in the art, including attaching proteinaceous binding sites using covalent bonding, UV crosslinking, and linking through an affinity set. As a non-limiting example, a proteinaceous binding site provided with a His-tag is coated onto nickel beads.

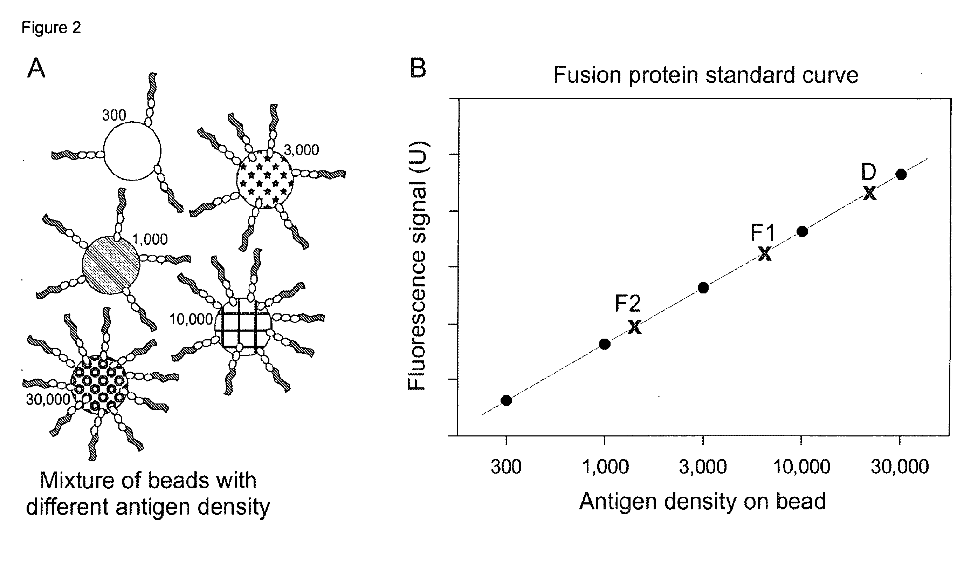

Figure 2 summarizes the mixture of the different beads and the standard curve which can be made based on the quantitation beads. This standard curve can be used for plotting the results of the fusion protein assay, thereby allowing the comparison between for example the results at diagnosis (D) and at multiple time points during follow-up (F1 and F2). In this way it becomes possible to accurately monitor the kinetics of the fusion protein levels and thereby the kinetics of the leukemic cells. Quantitative control analysis (C) is advantageously used in combination with assaying for a specific tumor-specific fusion protein during patient monitoring. However, also at the earlier step of diagnosis it may be useful to obtain information about the actual quantity of the fusion protein present prior to treatment. Said quantity is preferably expressed as amount of fusion protein per leukemic cell or per blood volume.

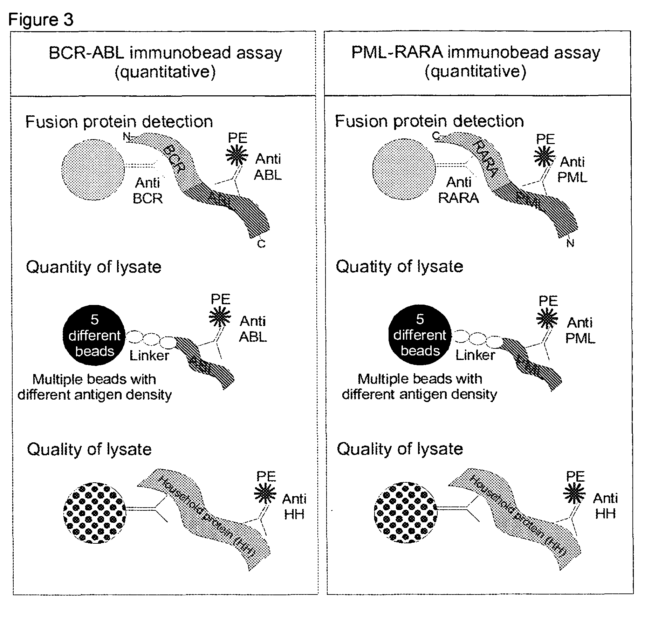

The quantitation beads can be run together with the appropriate immunobead fusion protein detection assay in a single-tube multiplex format (see Figure 3 for BCR-ABL and PML-RARA). Alternatively, it is possible to use the quantitation beads in a separate tube, for instance in a tube run parallel to multiple patient samples that are analyzed with respect to one or more tumor-specific fusion proteins.

The multiplexing possibility of a method of the invention allows for the combination of the fusion protein bead, the quality control bead (household protein bead) and the quantitation beads (see Figure 3).

The presented system is easy to establish and offers customizable and individual setup. The beads can be used for single analyte quantification or for multiplex arrays. Large quantities of distinctly colored microspheres can be manufactured at a very low cost. All components for the array can be deposited for months and can be assembled in a short time. The prepared multiplex bead array can be stored in a refrigerator and is stable for several weeks. Furthermore, arrays can be enlarged by encoding the bead subpopulations with templates of different diameters and by introducing additional fluorescence markers within the polyelectrolyte layers (e.g., Cy5). For instance, the standard bead technology marketed by Luminex has a hundred different beads on the basis of a mixture of two fluorochromes.

The sensitivity of the array could be further increased, e.g., by use of phycoerythrin (PE)-labeled secondary antibodies with a superior signal-to-noise ratio. Because the signal intensities increase with decreasing bead numbers at a given amount of antibodies, the performance of the system can be further optimized by adjustment for a proper particle-to-antibody ratio.

A further aspect of the invention relates to reducing the incidence of a false-negative outcome of a bead-based assay for detecting a tumor-specific fusion protein. This is achieved by means that increase the chance that a tumor-specific fusion protein which was originally present in a cell is actually being detected in the cell lysate that is subjected to a catching/detection assay. The inventors realized that several tumor-specific fusion proteins, such as PML-RARA, AML-ETO and TEL-AML1, are DNA binding molecules themselves or are involved in transcription factor protein complexes. In traditional cellular lysis methods, these nuclear proteins are not easily released from the nucleus and from the DNA bound protein complexes. Extra efforts are needed to release these fusion proteins in order to detect them with the immunobead assay. It is known in the art to release nuclear proteins by sonication. However, sonication has several drawbacks. First, it needs special laboratory facilities. Second, traditional sonicators use a probe that is directly in contact with the biological sample. This has major drawbacks in terms of reproducibility as the sonication energy depends on the depth of the sonication probe in the liquid. This hampers standardized transfer of protocols between (diagnostic) labs. Moreover, the probe system is tedious to work with, produces foam, and only one sample can be treated at a time. Also contamination between different samples is also frequently experienced. Of particular importance for the detection of tumor-specific fusion proteins, the present inventors found that a sonication step is preferably to be avoided in a method for detecting fusion proteins. For instance, the cytosolic fusion protein BCR-ABL was detected at a very low level when a lysate known to be positive for BCR-ABL was sonicated prior to analysis by a catching/detection assay. Without wishing to be bound by theory, it is thought that sonication causes (e.g. by denaturation) structural changes within the protein, thereby reducing its affinity to a specific (antibody) probe. Thus, sonication is unsuitable for use in a (multiplex) immunobead assay that aims to detect any tumor-specific fusion protein, irrespective of whether it is localized in the cytosol, the nucleus or any other cellular compartment.

It was surprisingly found that this problem can be overcome by means of a nuclease treatment, more preferably an endonuclease treatment, even more preferably a DNAse treatment, to release or liberate nuclear fusion proteins from nucleic acids and thereby making them more accessible for recognition by and binding to a catching/detection probe set. Accordingly, a method is decribed herein for detecting a fusion protein in a sample comprising cells, comprising a cell lysate comprising a nuclease and contacting the cell lysate with at least a bead-bound catching probe and a fluorochrome-conjugated detection probe directed against the at least one fusion protein, each probe capable of recognizing a binding site positioned at opposite sides of the fusion region of said fusion protein, and determining binding of said probes to said fusion protein by flow cytometry. Or in other words, we present a method for detecting a fusion protein in a sample comprising cells, comprising preparing a lysate of said cells and contacting the cellular lysate with at least a bead-bound catching probe and a fluorochrome-conjugated detection probe directed against the at least one fusion protein, each probe capable of recognizing a binding site positioned at opposite sides of the fusion region of said fusion protein, and determining binding of said probes to said fusion protein by flow cytometry, wherein preparing said cellular lysate comprises the use of a nuclease, preferably an endonuclease.

Several nucleases have been tested for detection of the DNA bound fusion proteins. Very good results were obtained by adding a non-specific endonuclease from Serratia marcescens to the lysis buffer for preparing a cell lysate, for instance Benzonase ® from Merck KGaA, Darmstadt. Benzonase® is a genetically engineered endonuclease, see US Patent 5,173,418 and EP Patent No. 0229866. A person skilled in the art can determine the concentration of endonuclease required to release a nuclear fusion protein. Preferably, Benzonase is used at a concentration of at least 10 mU/ml, preferably at least 20 mU/ml, such as 25 mU/ml or higher.

The Benzonase protocol was tested on several other fusion protein assays (e.g. ABL-BCR). These experiments demonstrated that, in contrast to the sonication procedure, the endonuclease treatment has no or only minor negative effects on the outcome of the immunobead assay for cytosolic tumor-specific fusion proteins (no or only a minor reduction of signal). Therefore, endonucleases like Benzonase may be advantageously included in the protocol for fusion protein detection, irrespective of the type or subcellular localization of the fusion protein. This is particularly important for the multiplex assays wherein various different tumor-specific fusion proteins are detected simultaneously in a single tube, such as in a multiplex assay.

The use of endonucleases is known in the art primarily with respect to subcellular fractionation, such as in methods for obtaining partial proteomes from the complete proteome of a cell preparation. US 7,262,283 discloses the use of Benzonase ® for the extraction of "insoluble" nuclear proteins (e.g., histones) in a method for the sequential production of a partial proteome enriched with cytosolic proteins, partial proteome enriched with membrane/organelle proteins, partial proteome enriched with proteins from the cell nucleus interior, partial proteome enriched with proteins of the cytoskeleton and of the nuclear matrix. The study of Martinez et al. (BMC Cancer. 2004; 4: 44) relates to fusion protein RUNX1-CBFA2T1 also known as AML1-ETOassociated with t(8;21)-positive acute myeloid leukemia. To obtain nuclear lysates for immunoblotting analysis of RUNX1-CBFA2T1, cells were washed in a lysis buffer comprising Benzonase. However, the prior art does not teach or suggest the use of an endonuclease in the preparation of a total cell lysate to be analyzed for a variety of tumor-specific fusion proteins using a (multiplex) bead-based catching/detection assay as disclosed herein.

A further solution to the problem of masked fusion protein detection as provided herein relates to the inhibition of protein degradation. As mentioned herein above, protease activity severely affects the integrity of the fusion proteins in a bead-based (immuno)detection assay. This problem is particularly prominent when (more) mature myeloid cells are present in the patient sample. Particularly in patient samples with high frequencies of granulocytes or CML cells, false-negative results can be obtained due to protein degradation. For example, BCR-ABL is readily detectable in precursor B-ALL cells and in tumor cell lines such as K562. However, in CML patients the detection of BCR-ABL is severely hampered, presumably due to myeloid proteases, such as cathepsin, protease 3 and elastase.

A series of protocol optimization experiments performed by the present inventors revealed that degradation of tumor-specific proteins was only effectively inhibited when protease inhibitors were added to the intact cells before cell lysis (i.e. in a preincubation step employing a non-lytic buffer) as well as during cell lysis (i.e. in the lysis buffer). This combined approach results in a surprisingly strong reduction of protease activity and thereby contributes to the prevention of false-negative results.

Accordingly, provided herein is a method for detecting a fusion protein in a sample comprising cells, comprising preparing a lysate of said cells and contacting the cellular lysate with at least a bead-bound catching probe and a fluorochrome-conjugated detection probe directed against the at least one fusion protein, each probe capable of recognizing a binding site positioned at opposite sides of the fusion region of said fusion protein, and determining binding of said probes to said fusion protein by flow cytometry, wherein preparing the cellular lysate comprises a treatment of intact (viable) cells with at least one cell permeable protease inhibitor, followed by lysis of said pretreated cells in a lysis buffer comprising one or more protease inhibitors. The cell sample comprises white blood cells, mononuclear cells, granulocytes or a mixture thereof. Preferably, the intact cells are incubated with at least one irreversible serine protease inhibitor. Very suitable serine protease inhibitors are phenylmethyl sulfonyl fluoride (PMSF) and 4-(2-Aminoethyl) benzenesulfonyl fluoride hydrochloride (AEBSF HCl). AEBSF is a water soluble, irreversible serine protease inhibitor with a molecular weight of 239.5 Da. It inhibits proteases like chymotrypsin, kallikrein, plasmin, thrombin, and trypsin. The specificity is similar to the inhibitor PMSF, nevertheless AEBSF is more stable at low pH values. PMSF is a serine protease inhibitor but it does not inhibit all serine proteases. PMSF is rapidly degraded in water and stock solutions are usually made up in anhydrous ethanol, isopropanol, corn oil, or DMSO.

The combined use of PMSF and AEBSF was found to result in a significant reduction of protease activity, particularly when combined with extra protease inhibitors in the lysis buffer (see Example 1). Therefore, preferably a combination of PMSF and AEBSF is used for pretreating intact cells. PMSF may be used at various concentrations, for instance 0.5 to 5 mM final, preferably 0.5-2 mM, such as around 1 mM final. AEBSF is typically used at a higher concentration, for example 5-50 mM, preferably 10-30 mM, such as around 20 mM.

The addition of extra protease inhibitors in the lysis buffer serves to further reduce the protease activity in the lysate. In a preferred embodiment, the lysis buffer is supplemented with a standard cocktail of several types of (broad spectrum) protease inhibitors. Since cells have different types of proteases, mixtures of different protease inhibitors are usually required to maintain and preserve cellular protein composition following cell lysis. A typical cocktail contains a mixture of water-soluble protease inhibitors with broad specificity for the inhibition of serine, cysteine, aspartic, and metalloproteases. These are well known in the art, and obtainable from various commercial sources. For example, the Protease Inhibitor Cocktail (BaculoGold ™) marketed by BD Biosciences Pharmingen may be used. This 50 x concentrated cocktail contains benzamidine HCl, phenanthroline, aprotinin, leupeptin, pepstatin and PMSF. Another commercially available protease mixture for supplementing the lysis buffer contains AEBSF, E-64, bestatin, leupeptin, aprotinin, and sodium EDTA. As will be understood, :he use of other combinations of protease inhibitors in a method of the invention is also encompassed.

Also described herein are kits for use in a method for detecting a fusion protein as disclosed. As will be understood, a kit can contain various components depending on the specific application (e.g. diagnosis, follow-up during treatment) desired.

the kit comprises,
- a probe set (A) comprising at least a first bead-bound fusion protein catching probe (A1) and a second fluorochrome-conjugated fusion protein detection probe (A2), each probe capable of recognizing a binding site positioned at opposite sides of the fusion region of said fusion protein,
- a probe set (B) comprising a bead-bound household protein catching probe (B1) and a fluorochrome-conjugated household protein detection probe (B2), each probe capable of recognizing binding sites positioned at distinct sites on a household protein expressed in said cell; and/or
- at least a first set of quantitation beads (C1), the beads being provided with a first known amount of binding sites for fusion protein detection probe (A2).

Referred probes are antibodies or functional fragments thereof. However, other types of specific binding molecules that can be conjugated to a bead and/or fluorochrome are also encompassed.

The kit may furthermore comprise a second set of quantitation beads (C2), the beads being provided with a second known amount of binding sites for probe (A2). Together with the first set of quantitation beads, this allows for the plotting of a standard curve. Preferably, the kit comprises at least three, more preferably at least four sets of quantitation beads, each set being provided with a different amount of binding sites for the fusion protein detection probe. It is of course to be understood that among the multiple sets of quantitation beads there is a significant difference in the amount of binding sites, e.g. at least a two- or three fold difference, between different sets of beads. Furthermore, it is preferred that the quantitation beads cover a range of binding sites that is or can be expected to cover the number of fusion protein to be detected in a sample. For example, a kit comprises five sets of beads, spanning a range of about 300 to about 30.000 binding sites per bead. For single tube assaying of the multiple sets of quantitation beads, it is preferred that the beads belonging to the different sets are distinguishable from each other, for instance on the basis of size and/or color. In that case, the kit may contain a mixture of two or more sets of quantitation beads. Most preferred are kits comprising differently colored sets of quantitation beads, like Luminex™ beads. Other useful kit components include buffers, lysing and/or washing reagents.

To allow for the detection of multiple tumor-specific fusion proteins, a kit preferably comprises at least two probe sets (A), each probe set being capable of specifically binding to and detecting a different tumor-specific fusion protein. For example, a kit comprises a bead-bound anti-BCR antibody and a fluorochrome-conjugated anti-ABL antibody for detecting BCR-ABL, as well as a bead-bound anti-RARA antibody and a fluorochrome-conjugated anti-PML antibody for detecting PML-RARA. Again, it is preferred that the different beads, i.e. the anti-BCR bead and the anti-PML bead, are distinguishable by flow cytometry. Of course, any combination of tumor-specific probe sets can be present in a kit. In one aspect, it comprises a probe set (A) directed against at least one, preferably at least two, tumor-specific fusion protein(s) selected from the group consisting of MLL-AF4, MLL-AF6, MLL-AF9, MLL-ENL, MLL-AF10, MLL-ELL, PML-RARA, PLZF-RARA, NPM-RARA, NUMA-RARA, NPM-ALK, TPM3-ALK, TFG-ALK, ATIC-ALK, EWS-FLI1, EWS-ERG, EWS-ETV1, AML1-ETO, PML-RARA, CBFB-MYH11, E2A-PBX1, BCR-ABL and TEL-AML1 (Mitelman et al., Nat. Rev. Cancer 2007;7:233-245)

Probe set (B) in a kit is directed against a household protein. For instance, it comprises a bead-bound catching antibody and a fluorochrome-conjugated antibody directed at distinct sites of a household protein selected from the group consisting of ABL, β2M, GUS, GADH, β-actin or others. To increase the reliability of the qualitative control analysis (B), it is preferred that the binding sites (epitopes) for the probe set (B) are sufficiently separated from each other, thereby covering as many protease sites as possible. For instance, they each reside in a stretch of amino acids that make up the terminal 25%, preferably 15%, most preferably the C- resp. N-terminal 10% of the household protein.

Also described herein is a kit for the flow cytometric detection of at least one tumor-specific fusion protein in a cell, the kit comprising a probe set (A) comprising at least a first bead-bound fusion protein catching probe (A1) and a second fluorochrome-conjugated fusion protein detection probe (A2), each probe capable of recognizing a binding site positioned at opposite sides of the fusion region of said fusion protein, wherein the kit furthermore comprises an endonuclease. As is described herein above, the endonuclease is advantageously used to enhance the detection of DNA-binding tumor-specific fusion proteins without the need for sonication. Preferably, the kit comprises a nonspecific endonuclease from *Serratia marcessens,* more preferably Benzonase ®

Also described is a kit comprising a first container comprising at least one cell permeable irreversible serine protease inhibitor, preferably phenylmethyl sulfonyl fluoride (PMSF) or 4-(2-Aminoethyl) benzenesulfonyl fluoride hydrochloride (AEBSF HCl), more preferably a combination of PMSF and AEBSF, and a second container comprising a protease inhibitor cocktail. The inhibitor cocktail may be present as part of a cell lysis buffer contained in the kit.

The sensitivity of a fusion protein detection assay can be improved by subjecting a cell lysate to a so-called "pre-clear step" disclosed in WO2005/015235. Accordingly, a kit may additionally comprise at least one bead-bound binding molecule specifically reactive with a native protein, wherein a fragment of said native protein is part of the tumor-specific protein to be detected and wherein said binding molecule is reactive with the native protein but not with the fusion protein. These so-called "preclear beads" are reactive with a fragment of BCR or ABL that is not present in the BCR-ABL fusion protein.

A kit is of use in many clinical diagnostic applications, in particular for the diagnosis, classification and/or monitoring of a disease. Provided is for instance a "Diagnosis kit" specifically designed for performing an immunobead assay on a patient sample suspected to contain a tumor-specific fusion protein but wherein the identity of said fusion protein has not yet been revealed. A Diagnosis kit typically comprises multiple probe sets A for simultaneous detection of multiple tumor-specific fusion proteins and qualitative control beads to evaluate the integrity of a cell lysate. The kit preferably also comprises PMSF, AEBSF and an endonuclease, like Benzonase™, for the preparation of a cell lysate wherein both the quality and quantity of the fusion proteins, if present, are maximized.

Also described is a "Monitoring kit" specifically designed for disease monitoring, e.g. for the follow-up of an MRD patient known to have a certain fusion protein and receiving therapy. A Monitoring kit typically comprises a probe set for detecting a defined fusion protein, like BCR-ABL, , quantitative control beads capable of binding the fusion protein detection probe (e.g. beads coated at a known density with a fragment of BCR that is recognized by fluorochrome-conjugated anti-BCR antibody). It preferably also comprises the appropriate preclear beads capable of depleting the native non-fused protein (e.g. BCR or ABL) to remove competing native protein from the lysate, thereby enhancing the detection sensitivity. Similar to the Diagnosis kit, it may furthermore comprise PMSF, AEBSF and an endonuclease, like Benzonase™.

### Legends to the figures

**Figure 1**: schematic representation of fusion protein detection and qualitative control analysis. Upper parts show detection of a tumor-specific fusion protein (panel A: BCR-ABL; panel B: RARA-PML) using a bead-bound catching probe directed against the BCR- resp. RARA- fragment of the fusion protein, and a fluorochrome (in this case PE)-conjugated detection probe capable of recognizing the ABL resp. PML-fragment of the fusion protein. In parallel, preferably in the same tube, the integrity of the lysate is evaluated using qualitative control beads capable of recognizing and catching a household protein (HH) and a fluorochrome (PE)-conjugated HH detection probe (panels 3 and 4). The use of distinguishable beads (in this case indicated by a different gray shading of the beads) used in panels 1-4 allows for the simultaneous detection of different fusion proteins and a household protein in a single tube.
**Figure 2**: panel A shows a schematic drawing of five sets of quantitation beads, each set of beads being provided with a different amount of binding sites (antigens) for the tumor-specific detection probe. Panel B shows an exemplary standard curve that can be obtained using the quantitation beads. This standard curve can be used for plotting the results of the fusion protein assay, thereby allowing the comparison between for example the results at diagnosis (D) and at multiple time points during follow-up (F1 and F2). In this way it becomes possible to accurately monitor the kinetics of the fusion protein levels and thereby the kinetics of the leukemic cells.
**Figure 3** : The quantitation beads can be run together with the involved immunobead assay (in this case BCR-ABL (panel A) and PML-RARA (panel B) detection) in a multiplex format. Alternatively, it is possible to use the quantitation beads in a separate tube in parallel to multiple patient samples that are analyzed with the involved immunobead assay. The quality of the lysate can be checked by detecting the amount of intact household protein. As in Figures 1 and 2, the different "grey colors" of the beads indicate that the different beads can be recognised during flow cytometry, while the fluorochrome of the detection antibodies can be the same.

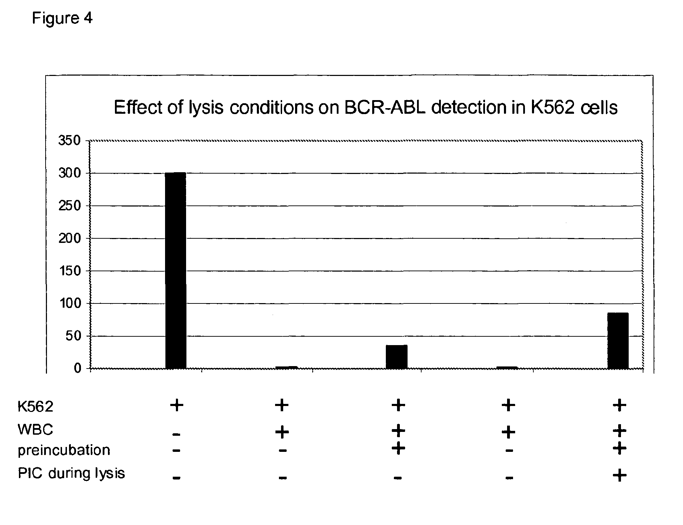

**Figure 4**: Effect of pretreating intact cells with protease inhibitors prior to cell lysis on the detection of BCR-ABL in K562 cells. For details, see Example 1. The Y-axis shows the BCR-ABL signal to noise ratio.

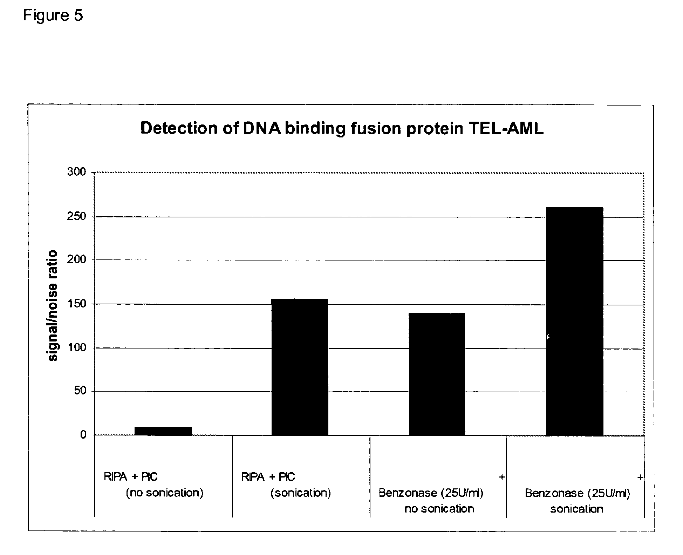

**Figure 5**: Effect of endonuclease treatment on the detection of a DNA-binding tumor-specific fusion protein TEL-AML. For details, see Example 2. The Y-axis shows the TEL-AML signal to noise ratio.

### Examples

### Example 1: Effect of Protease inhibitors (pre-treatment and/or during lysis) on BCR-ABL fusion protein detection.

**Cells**: K562 is a Ph+ cell line obtained from a CML patient during blast crisis. The cell line expresses the p210 isoform of the BCR-ABL fusion protein. 697, is a protease negative, Ph- leukemic cell line that contains the (t(1;19)) translocation. Total white blood cells (WBC) were obtained from whole blood of a healthy donor after NH4CI lysis of the erythrocytes. Blood was collected after informed consent of the healthy donor.

**Test samples**: To mimic a leukemic sample, cells of the Ph+ positive cell line K562 were mixed with WBC of a healthy donor (containing a large amount of proteases). As a control K562 cells were mixed in a 1:4 ratio with cells of the Ph- cell line 697, which contains only little protease activity. Prior to lysis, the still intact cells were incubated on in ice in the presence or absence of cell permeable protease inhibitors. Hereafter the cells were resuspended in a lysis buffer with or without protease inhibitors (PIC). After incubation on ice the cell lysate was spun down and the supernatant was used for a bead assay which detects the presence of the intact BCR-ABL fusion protein

**Pretreatment of the cells with cell permeable protease inhibitors:** To inhibit protease activity during cell lysis, intact cells were pretreated with cell permeable protease inhibitors. Pelleted cells were resuspended (maximum 107 cells/ml) in 1 ml pretreatment buffer supplemented with 20 mM AEBSF (4-(2-Aminoethyl)benzenesulfonyl fluoride hydrochloride, Sigma Aldrich, Zwijndrecht, The Netherlands) and 1or 5 mM PMSF (Phenylmethanesulfonyl fluoride, Sigma Aldrich) in PBS) and incubated for 10 minutes on ice. Hereafter the cells were spun down for 5 minutes at 520 G at 4°C.

**Generation of a cell lysate**: The pelleted cells were resuspended (25 million cells/ml, unless stated otherwise) in RIPA buffer (50 mM Tris HCL pH 7.4, 150 mM NaCl, 1%NP40, 0.5% Sodiumdeoxycholate, 1 mM EDTA, 0.1% SDS and 0.01% NaN3) in the presence or absence of a protease inhibitor cocktail (PIC, Sigma Aldrich, product nr. P2714). After 30 minutes on ice, the lysate was spun down to remove cell debris and the supernatant of the lysate was used in a flow cytometric bead assay.

**Bead Assay**: To a filterplate (Millipore), 50 µl of lysate, 25 µl beads provided with anti-BCR antibody (catching probe) beads and 50 µl PE-conjugated anti-ABL antibody (detection probe) in PBS/1% BSA were added (6000 beads in total) and incubated for two hours at room temperature while shaking. The filterplate was washed three times with PBS/1% BSA and drained. Subsequently, 1000 beads were acquired on a FACS Canto II flow cytometer (BD Biosciences).

For all different incubation conditions, the BCR-ABL bead assay was performed according to the standard protocol. For quantification the ratio was calculated between the mean fluorescence signal detected in the lysates of the K562 cells treated with the various incubation conditions and the mean fluorescence signal obtained when the assay (including pre-treatment, lysis and bead assay) is performed on a cell lysate of pure WBC of a healthy individual (background value).

**Results** Figure 4 shows that BCR-ABL is readily detectable in a cell lysate of protease poor K562 cells, even if no protease inhibitors are used. In contrast, lysis of K562 cells in the presence of granulocyte-rich WBC's results in a nearly complete loss of the detected signal. Preincubation of this cell mixture with AEBSF and PMSF partially restores the signal, whereas the mere addition of a protease inhibitor cocktail to the lysis buffer has no effect. Surprisingly, the combination of intact cell pretreatment and supplementing lysis buffer with the cocktail has a synergistic effect on the detection signal. Whereas the BCR-ABL signal is not fully restored to the level obtained in the absence of protease-rich cells, it is sufficient for a reliable assay outcome.

### Example 2: Endonuclease treatment can replace sonication.

The tumor-specific fusion protein TEL-AML is a DNA binding protein and can only be detected by a TEL-AML specific bead assays after sonication of the lysate. Since this procedure is not applicable in all standard diagnostic laboratories, the use of DNAses to digest DNA and make the TEL-AML (and other) fusion proteins accessible for detection was investigated.

**Cells**: REH t(12;12) is a cell line in which the oncogenic fusion protein TEL-AML is expressed.

**Generation of a cell lysate**: The pelleted cells (25 million cells/ml RIPA) were resuspended in RIPA buffer (50 mM Tris HCL pH 7.4, 150 mM NaCl, 1%NP40, 0.5% Sodiumdeoxycholate, 1 mM EDTA, 0.1% SDS and 0.01% NaN₃) to which a protease inhibitor cocktail (PIC, Sigma Aldrich) is added in the presence or absence of the non-specific endonuclease Benzonase™ (Novagen, Merck KGaA Darmstadt, Germany). Part of the lysates were sonicated and after 15 minutes incubation on ice, the lysate was spun down to remove cell debris and the supernatant was used in a cytometric bead assay.

### Test samples:

REH cells were spun down and the pelleted cells were resuspended in RIPA lysis buffer with added protease inhibitors (PIC). Here after
**Sample 1**: 15 minutes incubation on ice
**Sample 2**: cells were sonicated, followed by incubation on ice for 15 minutes
**Sample 3**: 25 U/ml of benzonase was added to the lysis buffer and cells were incubated for 15 minutes on ice.
**Sample 4**: 25 U/ml of benzonase was added to the lysis buffer, cells were sonicated and incubated for 15 minutes on ice.

After incubation, the various lysates were spun down and the supernatants were subjected to a TEL-AML specific catching/detection bead assay performed under standard conditions (see Example 1).

For quantification of the TEL-AML detection signal, the ratio was calculated between the mean fluorescence signal detected for the various cell lysates and the background value. The background value is the mean fluorescence signal obtained when the assay (including pre-treatment, lysis and bead assay) is performed on a lysate of a cell line known to be negative for the TEL-AML fusion protein.

**Results**: Figure 5 demonstrates that only a very low TEL-AML signal is detected in a lysate prepared using a lysis buffer with protease inhibitors only. Sonication of the lysate dramatically enhances the TEL-AML signal. Supplementing the lysis buffer with an endonuclease has essentially the same effect. The endonuclease treatment combined with sonication even further enhances the signal. However, the signal-to-noise-ratio obtained with endonuclease alone is more than sufficient for a reliable assay outcome. Sonication is preferably to be avoided in case also one or more cytosolic fusion proteins, such as BCR-ABL, are to be detected in the lysate.

## Claims

1. A method for detecting a tumor-specific fusion protein, said method comprising the steps of
A) a fusion protein analysis wherein a lysate of cells suspected to contain at least one tumor-specific fusion protein is contacted with at least a bead-bound catching probe and a fluorochrome-conjugated detection probe directed against the at least one fusion protein, each probe capable of recognizing a binding site positioned at opposite sides of the fusion region of said fusion protein, and determining binding of said probes to said fusion protein by flow cytometry,
B) subjecting the lysate to a qualitative control analysis comprising the step of contacting the lysate with at least a bead-bound catching probe and a fluorochrome-conjugated detection probe directed against a household protein known to be present in the cells, each probe capable of recognizing distinct binding sites of said household protein, and determining binding of said probes to said household protein by flow cytometry to determine whether the lysate of cells is of sufficient quality in terms of protein integrity; and
C) a quantitative control analysis comprising the step of contacting the fluorochrome-conjugated fusion protein detection probe as used in step (A) with at least a first set of quantitation beads being provided with a first known amount of binding sites for the fluorochrome-conjugated fusion protein detection probe, and measuring by flow cytometry the fluorescence signal that is associated with said known amount of binding sites to determine the amount of fusion protein detected in step (A).

2. The method according to claim 1, wherein the steps of fusion protein analysis (A) and control analysis (B) and/or (C) are performed simultaneously in a single tube using beads that are distinguishable during flow cytometric detection.

3. The method according to claims 1 or 2, wherein the bead-bound catching probe and a fluorochrome-conjugated detection probe directed against a household protein used in qualitative control analysis step (B) are capable of recognizing, respectively, an N-terminal and a C-terminal binding site of said household protein, or, respectively, a C-terminal and a N-terminal binding site of said household protein.

4. Method according to any one of the preceding claims, wherein said household protein is selected from the group consisting of ABL, β2M, MGUS, GAPDH, actin and β-actin.

5. Method according to any one of the preceding claims, wherein quantitative control analysis (C) comprises the use of at least a first set of quantitation beads comprising a first known amount of binding sites and a second set of quantitation beads comprising a second known amount of said binding sites, to allow the plotting of a standard curve.

6. Method according to any one of the preceding claims, wherein the step of fusion protein analysis (A) is preceded by depleting the lysate of at least one native protein that can compete with a tumor-specific fusion protein for binding to a probe used in protein analysis (A), wherein said depleting comprises contacting the lysate with at least one bead-bound binding molecule that is reactive with said native protein, wherein a fragment of said native protein is part of a tumor-specific protein to be detected and wherein said binding molecule is reactive with said native protein but not with the fusion protein.

7. Method according to any one of the preceding claims, comprising detecting at least two different tumor-specific fusion proteins simultaneously using a specific set of bead-bound catching probes and fluorochrome-conjugated detection probes for each of the fusion proteins.

8. Method according to any one of the preceding claims, comprising detection of one or more, preferably at least two, tumor-specific proteins selected from the group consisting of MLL-AF4, MLL-AF6, MLL-AF9, MLL-ENL, MLL-AF10, MLL-ELL, PML-RARA, PLZF-RARA, NPM-RARA, NUMA-RARA, NPM-ALK, TPM3-ALK, TFG-ALK, ATIC-ALK, EWS-FLI1, EWS-ERG, EWS-ETV1, AML1-ETO, PML-RARA, CBFB-MYH11, E2A-PBX1, BCR-ABL and TEL-AML1.

## Patentansprüche

1. Verfahren zum Nachweis eines tumorspezifischen Fusionsproteins, mit den folgenden Verfahrensschritten:
A) Fusionsproteinanalyse, wobei man ein Lysat von Zellen, von dem vermutet wird, dass es wenigstens ein tumorspezifisches Fusionsprotein enthält, mit wenigstens einer an Kügelchen gebundenen Fängersonde und einer gegen das wenigstens eine Fusionsprotein gerichteten Fluorochrom-konjugierten Nachweissonde in Kontakt bringt, wobei die Sonden jeweils in der Lage sind, eine auf entgegengesetzten Seiten des Fusionsbereichs des Fusionsproteins gelegene Bindungsstelle zu erkennen, und Bestimmen der Bindung der Sonden an das Fusionsprotein mittels Durchflusszytometrie;
B) qualitative Kontrollanalyse des Lysats, wobei man in einem Schritt das Lysat mit wenigstens einer an Kügelchen gebundenen Fängersonde und einer gegen ein Haushaltsprotein, von dem man weiß, dass es in den Zellen vorhanden ist, gerichteten Fluorochrom-konjugierten Nachweissonde in Kontakt bringt, wobei die Sonden jeweils in der Lage sind, unterschiedliche Bindungsstellen des Haushaltsproteins zu erkennen, und die Bindung der Sonden an das Haushaltsprotein mittels Durchflusszytometrie bestimmt, um zu bestimmen, ob das Lysat von Zellen von hinreichender Qualität hinsichtlich Proteinintegrität ist; und
C) quantitative Kontrollanalyse, bei der man in einem Schritt die Fluorochrom-konjugierte Fusionsproteinnachweissonde, wie sie in Schritt (A) verwendet wird, mit wenigstens einem ersten Satz Quantitätsbestimmungskügelchen, die mit einer ersten bekannten Menge an Bindungsstellen für die Fluorochrom-konjugierte Fusionsproteinnachweissonde versehen sind, in Kontakt bringt und das Fluoreszenzsignal, das mit der ersten bekannten Menge an Bindungsstellen assoziiert ist, mittels Durchflusszytometrie misst, um die Menge an in Schritt (A) nachgewiesenem Fusionsprotein zu bestimmen.

2. Verfahren nach Anspruch 1, wobei die Schritte Fusionsproteinanalyse (A) und Kontrollanalyse (B) und/oder (C) gleichzeitig in einem einzigen Röhrchen unter Verwendung von Kügelchen, die während des durchflusszytometrischen Nachweises unterscheidbar sind, erfolgen.

3. Verfahren nach Anspruch 1 oder 2, wobei die an Kügelchen gebundene Fängersonde und eine gegen ein Haushaltsprotein gerichtete Fluorochrom-konjugierte Nachweissonde, die im qualitativen Kontrollanalyseschritt (B) verwendet werden, in der Lage sind, eine N-terminale bzw. eine C-terminale Bindungsstelle des Haushaltsproteins oder eine C-terminale bzw. eine N-terminale Bindungsstelle des Haushaltsproteins zu erkennen.

4. Verfahren nach einem der vorhergehenden Ansprüche, wobei das Haushaltsprotein ausgewählt ist aus der Gruppe ABL, β2M, MGUS, GAPDH, Actin und β-Actin.

5. Verfahren nach einem der vorhergehenden Ansprüche, wobei die quantitative Kontrollanalyse (C) die Verwendung wenigstens eines ersten Satzes Quantitätsbestimmungskügelchen mit einer ersten bekannten Menge an Bindungsstellen und eines zweiten Satzes Quantitätsbestimmungskügelchen mit einer zweiten bekannten Menge der Bindungsstellen umfasst, um die graphische Auftragung einer Standardkurve zu gestatten.

6. Verfahren nach einem der vorhergehenden Ansprüche, wobei dem Schritt der Fusionsproteinanalyse (A) die Abreicherung des Lysats an wenigstens einem nativen Protein, das mit einem tumorspezifischen Fusionsprotein um die Bindung an eine bei der Proteinanalyse (A) verwendete Sonde konkurrieren kann, vorausgeht, wobei man bei der Abreicherung das Lysat mit wenigstens einem an Kügelchen gebundenen Bindungsmolekül, das mit dem nativen Protein reaktiv ist, in Kontakt bringt, wobei ein Fragment des nativen Proteins Teil eines nachzuweisenden tumorspezifischen Proteins ist und wobei das Bindungsmolekül mit dem nativen Protein, jedoch nicht mit dem Fusionsprotein reaktiv ist.

7. Verfahren nach einem der vorhergehenden Ansprüche, bei dem man wenigstens zwei unterschiedliche tumorspezifische Fusionsproteine gleichzeitig unter Verwendung eines spezifischen Satzes an Kügelchen gebundender Fängersonden und Fluorochrom-konjugierter Nachweissonden für jedes der Fusionsproteine nachweist.

8. Verfahren nach einem der vorhergehenden Ansprüche, umfassend den Nachweis eines oder mehrerer, vorzugsweise wenigstens zwei, tumorspezifischer Proteine, ausgewählt aus der Gruppe MLL-AF4, MLL-AF6, MLL-AF9, MLL-ENL, MLL-AF10, MLL-ELL, PML-RARA, PLZF-RARA, NPM-RARA, NUMA-RARA, NPM-ALK, TPM3-ALK, TFG-ALK, ATIC-ALK, EWS-FLI1, EWS-ERG, EWS-ETV1, AML1-ETO, PML-RARA, CBFB-MYH11, E2A-PBX1, BCR-ABL und TEL-AML1.

## Revendications

1. Procédé pour détecter une protéine de fusion spécifique d'une tumeur, ledit procédé comprenant les étapes de
A) une analyse de protéine de fusion dans laquelle un lysat de cellules suspectées de contenir au moins une protéine de fusion spécifique d'une tumeur est mis en contact avec au moins une sonde de capture liée à une bille et une sonde de détection conjuguée à un fluorochrome dirigée contre l'au moins une protéine de fusion, chaque sonde étant capable de reconnaître un site de liaison positionné à des côtés opposés de la région de fusion de ladite protéine de fusion, et détermination de la liaison desdites sondes à ladite protéine de fusion par cytométrie en flux,
B) soumettre le lysat à une analyse de contrôle qualitatif comprenant l'étape de mise en contact du lysat avec au moins une sonde de capture liée à une bille et une sonde de détection conjuguée à un fluorochrome dirigée contre une protéine domestique connue comme étant présente dans les cellules, chaque sonde étant capable de reconnaître des sites de liaison distincts de ladite protéine domestique, et détermination de la liaison desdites sondes à ladite protéine domestique par cytométrie en flux pour déterminer si le lysat de cellules est de qualité suffisante en termes d'intégrité de protéine ; et
C) une analyse de contrôle quantitatif comprenant l'étape de mise en contact de la sonde de détection de protéine de fusion conjuguée à un fluorochrome telle qu'utilisée dans l'étape (A) avec au moins un premier ensemble de billes de quantification étant pourvues d'une première quantité connue de sites de liaison pour la sonde de détection de protéine de fusion conjuguée à un fluorochrome, et la mesure par cytométrie en flux du signal de fluorescence qui est associé à ladite quantité connue de sites de liaison pour déterminer la quantité de protéine de fusion détectée dans l'étape (A).

2. Procédé selon la revendication 1, dans lequel les étapes d'analyse de protéine de fusion (A) et d'analyse de contrôle (B) et/ou (C) sont effectuées simultanément dans un tube unique en utilisant des billes qui peuvent être distinguées pendant la détection par cytométrie en flux.

3. Procédé selon les revendications 1 ou 2, dans lequel la sonde de capture liée à une bille et une sonde de détection conjuguée à un fluorochrome dirigée contre une protéine domestique utilisée dans l'étape d'analyse de contrôle qualitatif (B) sont capables de reconnaître, respectivement, un site de liaison N-terminal et C-terminal de ladite protéine domestique, ou, respectivement, un site de liaison N-terminal et C-terminal de ladite protéine domestique.

4. Procédé selon l'une quelconque des revendications précédentes, dans lequel ladite protéine domestique est choisie dans le groupe constitué de ABL, β2M, MGUS, GAPDH, l'actine et la β-actine.

5. Procédé selon l'une quelconque des revendications précédentes, dans lequel l'analyse de contrôle quantitatif (C) comprend l'utilisation d'au moins un premier ensemble de billes de quantification comprenant une première quantité connue de sites de liaison et un deuxième ensemble de billes de quantification comprenant une deuxième quantité connue desdits sites de liaison, pour permettre la représentation graphique d'une courbe d'étalonnage.

6. Procédé selon l'une quelconque des revendications précédentes, dans lequel l'étape d'analyse de protéine de fusion (A) est précédée par la déplétion du lysat d'au moins une protéine native qui peut entrer en compétition avec une protéine de fusion spécifique d'une tumeur pour liaison à une sonde utilisée dans l'analyse de protéine (A), où ladite déplétion comprend la mise en contact du lysat avec au moins une molécule de liaison liée à une bille qui est réactive avec ladite protéine native, où un fragment de ladite protéine native fait partie d'une protéine spécifique à une tumeur à détecter et où ladite molécule de liaison est réactive avec ladite protéine native mais pas avec la protéine de fusion.

7. Procédé selon l'une quelconque des revendications précédentes, comprenant la détection d'au moins deux protéines de fusion spécifique d'une tumeur différentes en utilisant simultanément un ensemble spécifique de sondes de capture liées à une bille et de sondes de détection conjuguées à un fluorochrome pour chacune des protéines de fusion.

8. Procédé selon l'une quelconque des revendications précédentes, comprenant la détection d'une ou plusieurs, de préférence au moins deux, protéines spécifiques d'une tumeur choisies dans le groupe constitué de MLL-AF4, MLL-AF6, MLL-AF9, MLL-ENL, MLL-AF10, MLL-ELL, PML-RARA, PLZF-RARA, NPM-RARA, NUMA-RARA, NPM-ALK, TPM3-ALK, TFG-ALK, ATIC-ALK, EWS-FLI1, EWS-ERG, EWS-ETV1, AML1-ETO, PML-RARA, CBFB-MYH11, E2A-PBX1, BCR-ABL et TEL-AML1.
